(19) Europäisches Patentamt
European Patent Office
Office européen des brevets

(11) Publication number: **0 174 181 B1**

(12) **EUROPEAN PATENT SPECIFICATION**

(45) Date of publication of patent specification: **03.04.91**

(51) Int. Cl.5: **C12P 1/06**, A61K 35/66, //(C12P1/04,C12R1:29)

(21) Application number: **85306244.6**

(22) Date of filing: **03.09.85**

(54) **Novel vasodilator and process for the preparation thereof.**

(30) Priority: **03.09.84 JP 184320/84**
**29.09.84 JP 184320/84**

(43) Date of publication of application:
**12.03.86 Bulletin 86/11**

(45) Publication of the grant of the patent:
**03.04.91 Bulletin 91/14**

(84) Designated Contracting States:
**DE FR GB IT**

(56) References cited:
**GB-A- 2 020 647**

(73) Proprietor: **KYOWA HAKKO KOGYO KABUSHIKI KAISHA**
**6-1, Ohte-machi 1-chome**
**Chiyoda-ku Tokyo 100(JP)**

(72) Inventor: **Kase, Hiroshi**
**3-35-18, Maehara-cho**
**Koganei-shi Tokyo-to(JP)**
Inventor: **Matsuda, Yuzuru**
**257 Congressional Lane**
**408 Rockville Maryland 2852(US)**
Inventor: **Kawamoto, Isao**
**1-21-2, Fujimino**
**Hiratsuka-shi Kanagawa-ken(JP)**
Inventor: **Asano, Kozo**
**3-9-10, Naka-machi**
**Machida-shi Tokyo-to(JP)**
Inventor: **Shrahata, Kunikatsu**
**4-11-5, Iwato-minami**
**Komae-shi Tokyo-to(JP)**
Inventor: **Yasuzawa, Tooru**
**1-21-2, Naruse**
**Machida-shi Tckyo-to(JP)**
Inventor: **Yamada, Koji**
**1-12-2 Asahi Machi Machida-shi**
**Tokyo-to(JP)**

(74) Representative: **Hildyard, Edward Martin et al**
**Frank B. Dehn & Co. Imperial House 15-19 Kingsway**
**London WC2B 6UZ(GB)**

EP 0 174 181 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention broadly relates to a physiologically active substance of microbiological origin and more particularly to a substance capable of dilating the arteries of humans and animals. The present invention further provides a process for the preparation of said substance.

Vasodilators, i.e. substances capable of dilating the vascular system, enhancing the peripheral circulation and/or decreasing the overall blood pressure, are useful, for example, in the treatment and amelioration of hypertension, angina pectoris, obstruction of peripheral veins, cardiac insufficiency and the like. Substances of microbiological origin which may be used as vasodilators have also been described in, for example, J. Antibiot., 35 , 151-156 (1982) and ibid., 35 , 157-163 (1982) wherein so-called WS-1228 substances are produced from Streptomyces aureofaciens.

Furthermore, antibiotics elaborated by microorganisms of the genus Micromonospora are also known. However, to our knowledge no vasodilators originating from microorganisms of this genus have been reported.

The present invention is based upon the discovery that a substance which we have isolated from the cultured broth of a microorganism of the genus Micromonospora is an effective vasodilator in mammals.

Therefore, in one aspect our invention provides a new substance, referred to herein as K-259-2, which is a vasodilator in mammals.

The new substance designated as K-259-2 has substantially the following physico-chemical characteristics:-

(1) Crystallises in reddish needles.

(2) Melting point:
140-145° C (decomp.). Becomes brownish at a temperature above about 140° C. Melting point indefinite.

(3) Specific rotation:
$[\alpha]_D^{20} = 0^\circ$ (c=0.33, $CH_3OH$)

(4) Solubility:
Readily soluble in acetic acid, soluble in methanol and water, and sparingly soluble in chloroform, ethyl acetate, acetone and ethanol.

(5) Colour reaction:
Positive in iodine and anisaldehyde reactions.

(6) Visible absorption spectrum:
As shown in Fig. 1 herein (82% methanol-water, v/v),

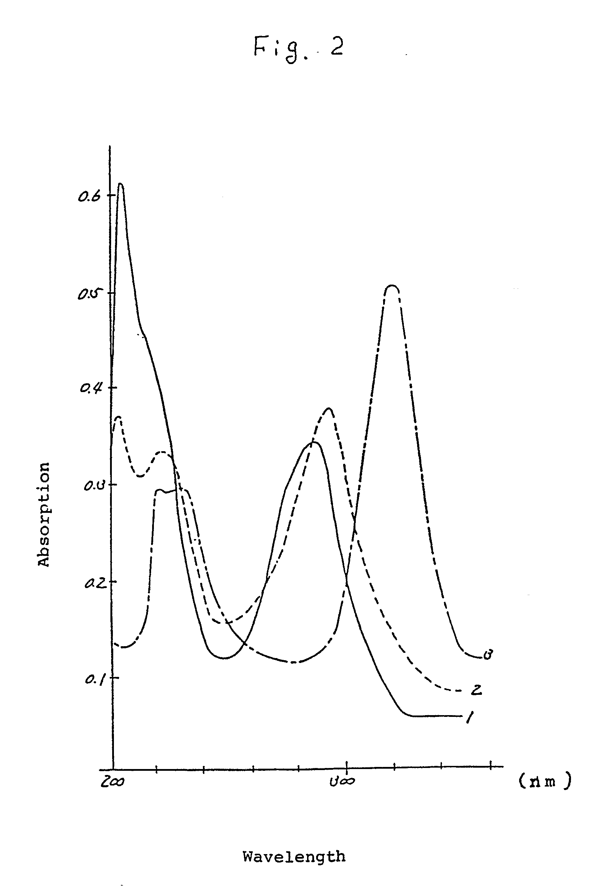

(7) Ultraviolet absorption spectrum:
As shown in Fig. 2 herein (82% methanol-water, v/v).

(8) Infrared absorption spectrum:
3425, 2960, 2924, 2852, 1626, 1562, 1442, 1386, 1326, 1259, 1227, 1176, 1157, 1108, 1052, 1023 $cm^{-1}$ (KBr disc method).

(9) Mass spectrum:
382 ($M^+$ ), 353, 334, 311, 308, 268.

(10) $^1H$-NMR spectrum (100 MHz, DMSO-$d_6$ + $CD_3OD$, ):
0.78(3H, t, J=7.3), 1.67(3H, br.d, J=5.9), ca. 1.7 (2H, m), 4.38(2H, br.s), ca. 5.1(1H, m), 6.36(1H, d, J=2.4), 6.93(1H, d, J=2.4), 7.36(1H, s).

(11) Molecular formula: $C_{21}H_{18}O_7$

(12) Molecular weight: 382

(13) Elementary analysis (%)

| | |
|---|---|
| Found: | H 4.50; C 65.53; N 0 |
| Calculated ($C_{21}H_{18}O_7$) | H 4.71; C 65.97; N 0; 0 29.32 |

(14) Rf values obtained by silica gel thin layer chromatography are shown in the following Table 1:-

Table 1

| | Solvent system | Rf value* |
|---|---|---|
| 1. | Chloroform/methanol/ethanol/water = 10: 4 : 4 : 2 (v/v) | 0.56 |
| 2. | n-butanol/ethanol/chloroform/ aqueous ammonia (28%) = 4 : 5 : 2 : 4 (v/v) | 0.35 |

* Thin layer...Kieselgel 60 (product of Merck & Co., Art 5631).

Developed at room temperature, ascending, one hour.

Detection was by absorption of UV light at 365 nm.

For further investigation, a trimethyl derivative of K-259-2 was produced by treating with diazomethane by the method of Example 2 described hereinafter. This derivative, which forms a further feature of our invention, has the following characteristics:-

(15-1) $^1$H-NMR spectrum (100 MHz, $CDCl_3$,$\delta$): 0.85(3H, t, J=6.6), 1.69(3H, br.d, J=6.7), 1.5-1.9(2H, m), 3.86(3H, s), 3.92(3H, s), 4.01 (3H, s), 4.21(2H, br.s), ca. 5.3 (1H, m), 6.71 (1H, d, J=2.7), 7.31(1H, d, J=2.7), 7.78(1H, s), 13.06 (1H, s)

(15-2) $^{13}$C-NMR spectrum (25 MHz, $CDCl_3$,$\delta$ c): 12.3; 13.6; 27.4; 32.8; 52.5; 56.0; 56.5; 106.7; 107.5; 108.1; 111.7; 117.8; 125.6; 132.0; 134.0; 137.5; 138.2; 144.1; 159.6; 165.3; 165.5; 167.1; 182.5; 188.1.

(15-3) Mass spectrum: m/Z 424 ($M^+$ )

(15-4) Molecular formula: $C_{24}H_{24}O_7$

(15-5) Infrared absorption spectrum ($CCl_4$): 3440, 3096, 3016, 2968, 2936, 2876, 2860, 1739, 1672, 1627, 1575, 1485, 1463, 1442, 1386, 1368, 1316, 1250, 1228, 1210, 1192, 1165, 1142, 1108, 1077, 1039, 1022, 993, 954, 936, 930, 892, 867, 850, 740, 704, 646, 617 $cm^{-1}$

By reference to the above-mentioned characteristics, it has been confirmed that K-259-2 is novel.

The acute toxicity of K-259-2 is very low. By reason of its vasodilator activity in mammals and its low toxicity, K-259-2 is of interest for the provision of propylactic or therapeutic vasodilating action. For such use the K-259-2 may be formulated with a physiologically acceptable carrier, excipient or adjuvant, using conventional methods of pharmacy.

According to another aspect of the present invention, there is provided a process for producing K-259-2, which comprises culturing a microorganism of the genus Micromonospora capable of producing K-259-2 in a suitable culture medium to accumulate the K-259-2 therein and recovering the K-259-2 from said medium.

Any and all microorganisms of the genus Micromono spora capable of producing K-259-2 may be used for the purpose of the present invention. Preferred strains for this purpose include, for example, Micromonospora sp. K-259 (FERM BP 569) isolated by us from the soil at Nagaizumi-cho, Mishima-shi, Shizuoka-ken, Japan. This strain was deposited with Bikoken (Fermentation Research Institute, Agency of Industrial Science and Technology, Japanese Government) on 18th July 1984 under the provisions of The Treaty of Budapest as FERN BP 569. The strain forms another feature of the present invention.

K-259 strain has the following mycological characteristics:

Morphological characteristics:

This strain grows moderately on various organic media and the growth is moderate or poor on synthetic media. Aerial mycelium is not formed on all agar plate media. Vegetative mycelia coloured orange to dark green or black grow up on yeast extract-malt extract agar medium and Hickey-Tresner agar medium. Single spores are formed at the upper ends of the simply branched vegetative mycelium, the spores being spherical and having a diameter of about 0.7 - 0.9 micron. Under the electron microscope, the surface of the spores is seen to be warty and neither flagellum nor sporangium are found. A brownish soluble pigment is only formed in yeast extract-malt extract agar medium.

Cultural characteristics on various media:

The following cultural characteristics were obtained by culturing the strain at 28° C for 2 weeks in each test. Colour name and hue number are expressed by reference to the classification shown in Colour Harmony Manual edited by Container Corporation of America.

When cultured on the following media, no aerial mycelium was formed. Soluble pigment was not produced except in yeast extract-malt extract agar medium.

In the following cultural characteristics, G denotes the growth, S and R denote respectively the surface and the reverse of vegetative mycelia.

(1) Sucrose-nitrate-agar medium;
G...moderate; S and R...black (17 pn)
(2) Glucose-asparagine-agar medium:
G...poor; S and R...black (19 pn)
(3) Glycerol-asparagine-agar medium (ISP No. 5):
G...poor; S and R...black olive (1 po)
(4) Starch-agar medium (ISP No. 4)
G...poor to moderate; S and R...bright orange (4 ga)
(5) Tyrosine-agar medium (ISP No. 7)
G...poor; S and R...black (15 po)
(6) Nutrient-agar medium
G...poor; S and R...copper (5 lc) to dark bottle green (22 pn)
(7) Yeast extract-malt extract-agar medium
G...good (raised); S and R...black (15 po); Soluble pigment...mustard brown (2 pl)
(8) Oatmeal-agar medium
G...moderate; S and R... burnt orange (5nc)
(9) Hickey-Tresner-agar medium
G...good (raised); S and R...golden brown (4 pi) to black (18 pn)
(10) Peptone-yeast extract-iron-agar medium
G...moderate; S and R...amber (4 pe)

Physiological characteristics:

The strain was cultured at 28° C for 3 weeks and then the following characteristics were determined with the exception that the growth temperature and the activity upon skim milk and cellulose were respectively detected 2 days and one month after the beginning of culturing.

(1) Utilisation of carbon sources:

Positive:

D-glucose, L-arabinose, D-xylose, D-fructose, L-rhamnose, sucrose and melibiose

Negative:

i-inositol, D-mannitol and raffinose

(2) Liquefaction of gelatin: negative

(3) Coagulation of skim milk and peptonization: positive

(4) Decomposition of cellulose: positive

(5) Hydrolysis of starch: positive

(6) Growth temperature range: 18 - 33°C,

optimal range 26 30°C

(7) Formation of melanoid pigment:negative

Strain K-259 is mesophilic. No aerial mycelium is formed. Single spores are formed at the upper ends of simply branched vegetative mycelium. Fragmentation of mycelium is not found. Analysis of the cell wall indicates the presence of 2,6-diamino-3-hydroxypimelic acid. Analysis of sugars in the whole cells indicates the presence of xylose and arabinose. From these facts, it has been found that this strain may be classified into the genus Micromonospora of the order Actinomycetales

Strain K-259 may be cultured in a conventional manner used for culturing strains of Actonomyces. Thus, any synthetic or organic medium may be used for culturing the strain of the present invention when it contains suitable amounts of assimilable carbon source, nitrogen source, inorganic substances and the like.

By way of example, it is preferred to use as the carbon source, glucose, fructose, sucrose, starch, dextrin, mannitol, maltose, molasses and other carbohydrates, citric acid, malic acid, acetic acid, fumaric acid and other organic acids, methanol, ethanol and other alcohols, methane, ethane, propane, n-paraffin and other hydrocarbons, glutamic acid and other amino acids and glycerol.

The nitrogen sources which may be used for the purpose of the present invention are examplified by ammonium salts of hydrochloric, sulfuric, nitric and phosphoric acids; aspartic acid, glutamine, cysteine, alanine and other amino acids; urea, peptone, meat extract, yeast extract, dried yeast, corn steep liquor, soybean powder, cotton seed cake, soybean casein, cazamino acid, Pharmamedia and the like.

Preferred inorganic substances which may be used for the purpose of the present invention include, for example, potassium dihydrogenphosphate, sodium dihydrogenphosphate, magnesium sulfate, ferrous sulfate, manganese sulfate, cobalt sulfate, zinc sulfate, calcium pantothenate, ammonium molybdate, aluminium potassium sulfate, barium carbonate, calcium carbonate, cobalt chloride, sodium chloride and the like.

If desired, it is possible to add to the medium, for example, vitamins such as thiamine and various other substances capable of promoting the growth of strain K-259 and/or promoting the production of the K-259-2. When an auxotrophic strain is cultured, it is necessary to supplement the medium with a suitable nutrient required for the growth of the strain used.

The fermentation may usually be effected under aerobic conditions, for example, by shaking or shaking with aeration at a temperature of 20 - 40°C and at a neutral pH. Culturing is usually discontinued when the concentration of the K-259-2 reaches a maximum, for example, 3-15 days after the beginning of culturing.

The K-259-2 may be isolated from the cultured broth in any convenient way, e.g. by methods conventionally used for isolating various known physiologically active substances from a cultured broth. Thus, the isolation of K-259-2 may be effected, for example, by removal of cells by filtration, centrifugation or the like, followed by adsorption and desorption of the active substances by column chromatography or thin layer chromatography or the like using, for example, adsorbing resins, silica gel, Silanised Silica-gel (product of Merck & Co.) alumina, cellulose, diatomaceous earth, magnesium silicate, gel filtration agent and the like.

K-259-2 may be isolated from the cultured broth, for example, in the following manner.

The cells are removed from the cultured broth, for example by filtration or centrifugation. The resultant filtrate or supernatant is treated, for example, with an adsorbing resin such as Diaion HP-10 (Mitsubishi Kasei Kogyo K.K., Japan) to absorb the active material onto the resin. The active material is eluted with a suitable solvent such as, for example, methanol, followed by removal of the solvent by evaporation under reduced pressure. To the resultant aqueous solution containing the active material is added a suitable solvent such as, for example, ethyl acetate, butyl acetate and the like, which is not miscible with water to extract the active material.

The extracted solution is concentrated under reduced pressure and then purified by repeated silica gel column chromatography. In this case, for example, a solvent system of butanol/ethanol/chloroform/concentrated- aqueous ammonia (4 : 5 : 2 : 4 v/v) may be used, followed by the use of a solvent system of, for example, chloroform/methanol/ethanol/water (10 : 4 : 4 : 2 v/v). The eluted fractions containing the K-259-2 are collected, then combined and concentrated under reduced pressure. The concentrated solution is subjected to chromatography using, for example, Sephadex LH-20 (Pharmacia Fine Chemicals AB., Sweden) and elution is carried out using methanol. The eluate is collected, combined and treated by silica gel chromatography using, for example, a solvent system of chloroform/methanol/ethanol/water (10 : 4 :4 : 2 v/v). The eluted fractions containing the K-259-2 product are collected, combined and concentrated under reduced pressure. The concentrated solution is subjected to reverse phase silica gel chromatography, for example using a solvent system of methanol/water (7 : 3 v/v). The eluate is collected, combined and concentrated under reduced pressure to obtain the K-259-2 as a crude powder. The crude product may be purified by recrystalization using a suitable solvent such as, for example, methanol, water or the like to obtain K-259-2 as crystalline needles.

In the above-mentioned process, the active substance may be assayed by silica gel thin layer chromatography, monitored by iodine reaction or by UV absorption at 365 nm.

The following non-limiting Examples illustrate the present invention.

## Example 1

Micromonospora sp. K-259 (FERM BP 569) was inoculated to a first seed medium [40 ml; glucose (1.0 g/dl), soluble starch (1.0 g/dl), meat extract (0.3 g/dl), yeast extract (0.5 g/dl), Bactotryptone, Difco., U.S.A. (0.5 g/dl) and calcium carbonate (0.2 g/dl) (pH 7.2 - 7.4)] in an Erlenmeyer flask (300 ml). Culturing was effected with shaking at 30°C for a period of time sufficient for growth of the strain. 40 ml of the resultant seed was inoculated to a second seed medium (400 ml) having the same composition as that described above, in a 2 1 Erlenmeyer flask and cultured with shaking at 28°C for 3 days. The resultant seed (1.2 1) was transferred to 18 1 of a fermentation medium in a 30 1 jar fermentator, the fermentation medium having the following composition:-

Glucose 0.5 g/dl; soluble starch 3.0 g/dl;
soybean powders 3.0 g/dl; corn steep liquor 0.5 g/dl; yeast extract 0.5 g/dl; calcium carbonate 0.3 g/dl (pH 7.2 - 7.4)

Culturing was effected with aeration and shaking at 30°C for 4 days to accumulate 5 mcg/ml of K-259-2 in the cultured broth. After completion of culturing, the cultured broth was continuously centrifuged (15000 r.p.m.) to obtain a supernatant, of which 70 1 was transferred to a column (2 1) packed with Diaion HP-10 to adsorb the K-259-2, followed by washing with methanol (30%, 6 1). The elution was effected with methanol (6 1). The eluted fractions were collected, combined and concentrated to 500 ml. After adjusting the pH to 2.0 with 2N hydrochloric acid, extraction was effected with ethyl acetate (1.5 1). The ethyl acetate layer was treated with anhydrous sodium sulfate and then concentrated to dryness to obtain an oily material (18 g). The oily material was transferred to a column packed with silica gel (2 1; Wako gel, Wako Junyaku K.K. Japan) equilibrated with a solvent system of butanol/ethanol/chloroform/ concentrated aqueous ammonia (4 : 5 : 2 : 4 v/v). Elution was effected with the same solvent system as that described above. The eluate was divided into fractions (each 18 g). The active material was present in Fraction Nos. 40 - 75 which were combined and concentrated to dryness under reduced pressure to obtain an oily material (about 4.0 g).

This oily material was transferred to a column packed with silica gel (1 1; Wako gel) equilibrated with a solvent system of chloroform/methanol/ethanol/ water (10 : 4 : 4 : 2 v/v). The elution was effected with the same solvent system as that described above. The eluate was divided into fractions (each 18 g). K-259-2 product was present in Fraction Nos. 12-40, which were combined and concentrated to dryness under reduced pressure to obtain an oily material (about 200 mg). The oily material was dissolved in methanol (5 ml) and transferred to a column packed with Sephadex LH-20 (200 ml, Pharmacia Fine Chemicals AB., Sweden). Methanol was used for elution. The eluate was divided into fractions (each 10 g). The K-259-2 was present in Fractions Nos. 18-44, which were combined and concentrated to dryness under reduced pressure to obtain an oily material (about 60 mg).

The oily material was transferred to a column packed with 100 ml of silica gel (Wako gel) equilibrated with a solvent system of chloroform/methanol/ethanol/ water (10 : 4 : 4 : 2 v/v). Elution was effected by using the same solvent system as that described above. The eluate was divided into fractions (each 5 g). The K-259-2 was present in Fractions Nos. 5-26, which were collected, combined and concentrated under reduced pressure to obtain reddish brown powders (about 30 mg). The powders were dissolved in methanol (about 3 ml) and absorbed onto Silanised Silica-gel RP-8 (size B, Art 11804, Merck AG., West Germany) equilibrated with a solvent system of methanol/water (7 : 3 v/v). Elution was effected with the same solvent system as that described above. The eluate was divided into fractions (each 10g). The K-259-2 was present in Fractions Nos. 30-64, which were then combined and concentrated to dryness under reduced pressure to obtain a red powder (about 25 mg). The powder was dissolved in methanol (about 5 ml).

After addition of water (3 ml), the solution was allowed to stand at 5°C for a period of about 7 days, yielding red needle crystals (21 mg).

In the above-mentioned purifying step, the K-259-2 was assayed by means of silica gel thin layer chromatography, followed by either the iodine reaction or visualisation under UV (365 nm).

The following Example 2 demonstrates the vasodilating activity of K-259-2. In this test, an excised animal artery was used to show the inhibition of the contraction of vascular tissue by K-259-2. Example 3 describes the preparation of a trimethyl derivative of K-259-2.

Example 2

1) Preparation of arterial test sample:

By a mid-line incision over the abdominal region, the superior mesenteric artery having a length of about 2-2.5 cm was cut out from the branch of the abdominal aorta of a mongrel rabbit (male; body weight 2-3 kg) to prepare a sample of artery in the form of a spiral having a width of 3-4 mm. The sample was ligated with silk threads at the both ends. The lower end was attached to a stationary bar and the upper end was connected to a high performance transducer (SB-1T, Nihon Koden K.K., Japan). The sample was hung down with an initial tension of 1.5 g. A peristaltic pump (Harbard 1210) was used to keep the sample at 36-38°C in an atmosphere of 95% oxygen containing 5% carbon dioxide. Surface perfusion of the sample was effected at a rate of 10 ml/min by the use of Krebs-Henseleit solution composed of NaCl (6.92 g/l), KCl (0.35 g/l), $MgSO_4.7H_2O$ (0.29 g/l), $CaCl_2$ (0.28 g/l), $KH_2PO_4$ (0.16 g/l), $NaHCO_3$ (2.1 g/l) and glucose (2.0 g/l).

After stabilizion for a period of 1-2 hours, the sample was used for experiment in the following manner.

2) As a vasoconstrictor, potassium chloride was used at a final concentration of 20 mM, supplied to the perfusion tubing from an inlet positioned very near the sample. The contractile reaction induced by surface perfusion was recorded on a polygraph (RM-45, Nihon Koden K.K., Japan) via the tension transducer substantially in the natural scale.

Then K-259-2 was dissolved in ethanol at a final concentration of 20 mg/ml, and the solution was suitably diluted with a nutrient solution to prepare sample solutions of the K-259-2. On each occasion, the administration of the sample solution was started 10 minutes before the beginning of the experiment and continued during the experiment.

3) Results are shown in Table 2.

Table 2

| Constrictor | Concentration K-259-2* | Contractile Inhibition (%) ** |
|---|---|---|
| Potassium Chloride | 3 | 15.4 |
| 20 mM | 10 | 45.5 |

Notes:-

* Final concentration, mcg/ml

** Inhibition rate (%) = (1 - A/B) X 100.
A=contractile rate in the presence of K-259-2
B=contractile rate in the absence of K-259-2

Table 2 indicates that K-259-2 was capable of inhibiting the contraction of the mesenteric arterial wall of rabbit and its activity was proportional to its concentration.

Example 3

In this experiment, 45 mg of K-259-2 obtained in Example 1 was dissolved in 5 ml of ethyl acetate. 2 ml of diazomethane ether solution was added and the mixture was stirred at room temperature for 0.5 hour. The solution was evaporated in vacuo and subjected to silica gel column chromatography. Elution was carried out using a solvent system of n-hexane/ethyl acetate (4 : 1 v/v). Fractions containing a trimethyl derivative of K-259-2 were combined and concentrated in vacuo to obtain 38 mg of a trimethyl derivative of K-259-2, having the physical characteristics given hereinbefore.

Drawings

Fig. 1 shows the visible absorption spectrum of K-259-2, in which traces (1), (2) and (3) denote respectively the absorption spectra in 0.1 N HCl/82% (v/v) aqueous methanol; 82% (v/v) aqueous methanol; and 0.1 N NaOH/82% (v/v) aqueous methanol. Fig. 2 shows the ultraviolet absorption spectrum of K-259-2 in which traces (1) to (3) were obtained in the solvent media defined above.

**Claims**

1. A substance having vasodilating activity in mammals, designated K-259-2, and having substantially the following physico-chemical characteristics:-
   (1) Crystallises in reddish needles.
   (2) Melting point:
   140-145 °C (decomp.). Becomes brownish at a temperature above about 140 °C. Melting point indefinite.
   (3) Specific rotation:
   $[\alpha]_D^{20} = 0^\circ$ ($c = 0.33$, $CH_3OH$)

(4) Solubility:
Readily soluble in acetic acid, soluble in methanol and water, and sparingly soluble in chloroform, ethyl acetate, acetone and ethanol.
(5) Colour reaction:
Positive in iodine and anisaldehyde reactions.
(6) Visible absorption spectrum:
As shown in Fig. 1 herein (82% methanol-water, v/v),
(7) Ultraviolet absorption spectrum:
As shown in Fig. 2 herein (82% methanol-water, v/v).
(8) Infrared absorption spectrum:
3425, 2960, 2924, 2852, 1626, 1562, 1442, 1386, 1326, 1259, 1227, 1176, 1157, 1108, 1052, 1023 $cm^{-1}$ (KBr disc method).
(9) Mass spectrum:
382 ($M^+$ ), 353, 334, 311, 308, 268.
(10) $^1H$-NMR spectrum (100 MHz, DMSO-$d_6$ + $CD_3OD$,$\delta$ ):
0.78(3H, t, J=7.3), 1.67(3H, br.d, J=5.9), ca. 1.7 (2H, m), 4.38(2H, br.s), ca.5.1(1H, m), 6.36(1H, d, J=2.4), 6.93(1H, d, J=2.4) , 7.36(1H, s).
(11) Molecular formula: $C_{21}H_{18}O_7$
(12) Molecular weight: 382
(13) Elementary analysis (%) Found:
H 4.50; C 65.53; N O
(14) Rf values by thin layer chromatography on silica gel:
0.56 (chloroform/methanol/ethanol/water = 10 : 4 : 4 : 2 Kieselgel, Merck) 0.35 (n-butanol/ethanol/chloroform/aqueous ammonia (28%) =4 : 5 : 2 : 4 v/v; Kieselgel, Merck).

**2.** The trimethyl derivative of K-259-2 as defined in claim 1, obtainable by methylation of K-259-2 by diazomethane.

**3.** A process for producing K-259-2 as defined in claim 1, which comprises culturing a microorganism of the genus Micromonospora capable of producing K-259-2 in a suitable culture medium to accumulate the K-259-2 therein and recovering the K-259-2 from said medium.

**4.** The process of claim 3 wherein said microorganism is Micromonospora sp. K-259 [FERM BP 569], or a mutant thereof capable of producing K-259-2 as defined in claim 1.

**5.** The strain Micromonospora sp. K-259 [FERM BP 569], and mutants thereof capable of producing K-259-2 as defined in claim 1, said strain being substantially biologically pure.

**6.** A vasodilating composition for human or veterinary use comprising an effective amount of a substance of claim 1 and a physiologically acceptable carrier, excipient or adjuvant.

**7.** The use of a substance of claim 1 to prepare a vasodilating composition for human or veterinary use.

## Revendications

**1.** Substance appelée K-259-2 exerçant une activité vasodilatatrice sur les mammifères et présentant principalement les caractéristiques physico-chimiques suivantes :
(1) Cristallise en aiguilles rougeâtres.
(2) Point de fusion :
140-145°C (décomposition). Devient brunâtre à une température au-dessus d'environ 140°C. Point de fusion indéfini.
(3) Rotation spécifique :
$[\alpha]_D^{20} = 0^\circ$ (c=0,33, $CH_3OH$)
(4) Solubilité :
très soluble dans l'acide acétique, soluble dans le méthanol et l'eau et peu soluble dans le chloroforme, l'acétate d'éthyle, l'acétone et l'éthanol.
(5) Réaction colorée :

réactions à l'iode et à l'anisaldéhyde positives.
(6) Spectre d'absorption visible :
Comme représenté sur la figure 1 annexée (méthanol aqueux à 82 % en volume).
(7) Spectre d'absorption ultraviolette :
comme représenté sur la figure 2 annexée (méthanol aqueux à 82 % en volume).
(8) Spectre d'absorption infrarouge :
3425, 2960, 2924, 2852, 1626, 1562, 1442, 1386, 1326, 1259, 1227, 1176, 1157, 1108, 1052, 1023 $cm^{-1}$ (méthode au disque de KBr).
(9) Spectre de masse :
382 ($M^+$), 353, 334, 311, 308, 268.
(10) Spectre de résonance magnétique des protons (100 MHz, DMSO-$d_6$ + $CD_3OD$, $\delta$) :
0,78 (3H, t, J=7,3), 1,67 (3H, large d, J=5,9), env. 1,7 (2H, m), 4,38 (2H, large s), env. 5,1 (1H, m), 6,36 (1H, d, J=2,4), 6,93 (1H, d, J=2,4), 7,36 (1H, s).
(11) Formule moléculaire : $C_{21}H_{18}O_7$.
(12) Poids moléculaire : 382
(13) Analyse élémentaire (%) :
Trouvé : H 4,50 ; C 65,53 ; N O.
(14) Valeurs de Rf dans la chromatographie sur couche mince sur gel de silice : 0,56 (chloroforme/méthanol/éthanol/eau = 10 : 4 : 4 : 2, gel de silice Kieselgel, Merck),
0,35 (n-butanol/éthanol/chloroforme/solution aqueuse d'ammoniac (28 %) = 4:5:2:4 en volume : gel de silice Kieselgel, Merck).

2. Le dérivé triméthylique de K-259-2 tel que défini dans la revendication 1, pouvant être obtenu par méthylation de K-259-2 au diazométhane.

3. Procédé de production de K-259-2, qui consiste à cultiver un micro-organisme du genre Micromonospora capable de produire du K-259-2 dans un milieu de culture convenable pour y accumuler le K-259-2, et à recueillir le K-259-2 dans ce milieu.

4. Procédé suivant la revendication 3, dans lequel ledit micro-organisme est Micromonospora sp. K-259 [FERM BP 569], ou un mutant de ce micro-organisme capable de produire du K-259-2 comme défini dans la revendication 1.

5. La souche Micromonospora sp. K-259 [FERM BP 569] et ses mutants producteurs de K-259-2 suivant la revendication 1, ladite souche étant très pure du point de vue biologique.

6. Composition vasodilatatrice pouvant être utilisée en médecine humaine ou en médecine vétérinaire, comprenant une quantité efficace d'une substance suivant la revendication 1 et un support, excipient ou adjuvant physiologiquement acceptable.

7. Utilisation d'une substance suivant la revendication 1 pour préparer une composition vasodilatatrice à usage en médecine humaine ou vétérinaire.

## Ansprüche

1. Substanz mit vasodilatorischer Wirkung bei Säugetieren, bezeichnet als K-259-2, und im wesentlichen mit den folgenden physikalisch-chemischen Kennzeichen:
(1) Kristallisiert in rötlichen Nadeln;
(2) Schmelzpunkt:
140-145 °C (Zersetzung); wird bräunlich bei einer Temperatur oberhalb von etwa 140 °C; Schmelzpunkt undefiniert;
(3) Spezifische Drehung:
$[\alpha]_D^{20} = 0°$ (c=0,33, $CH_3OH$)
(4) Löslichkeit:
leicht löslich in Essigsäure, löslich in Methanol und Wasser, und schwach löslich in Chloroform, Ethylacetat, Aceton und Ethanol;
(5) Farbreaktion:

Positiv bei Iod- und Anisaldehydreaktionen;
(6) Sichtbares Absorptionsspektrum:
wie in beiliegender Fig. 1 gezeigt (82% Methanol-Wasser, V/V);
(7) Ultraviolett-Absorptionsspektrum:
wie in beiliegender Fig. 2 gezeigt (82% Methanol-Wasser, V/V);
(8) Infrarot-Absorptionsspektrum:
3425, 2960, 2924, 2852, 1626, 1562, 1442, 1386, 1326, 1259, 1227, 1176, 1157, 1108, 1052, 1023 $cm^{-1}$ (KBR-Pastille);
(9) Massenspektrum:
382 ($M^+$), 353, 334, 311, 308, 268;
(10) $^1H$-NMR-Spektrum (100 MHz, DMSO-$d_6$ + $CD_3OD$, $\delta$):
0,78 (3H, t, J=7,3), 1,67 (3H, br.d, J=5,9), ca. 1,7 (2H, m), 4,38 (2H, br.s), ca. 5,1(1H, m), 6,36(1H, d, J=2,4), 6,93(1H, d, J=2,4), 7,36(1H,s);
(11) Summenformel: $C_{21}H_{18}O_7$;
(12) Molekulargewicht: 382;
(13) Elementaranalyse gefunden (%):
H 4,50; C 65,53; N O;
(14) Rf-Werte der Dünnschichtchromatographie an Silikagel:
0,56 (Chloroform/Methanol/Ethanol/Wasser = 10 : 4 : 4 : 2 Kieselgel, Merck); 0,35 (n-Butanol/Ethanol/Chloroform/wässeriges Ammoniak (28%) = 4 : 5 : 2 : 4 V/V; Kieselgel, Merck);

**2.** Trimethylderivat von gemäß Anspruch 1 definiertem K-259-2, erhältlich durch Methylierung von K-259-2 mit Diazomethan.

**3.** Verfahren zur Herstellung von gemäß Anspruch I definiertem K-259-2, wobei man einen Mikroorganismus der Gattung Micromonospora kultiviert, der zur Erzeugung und Anreicherung von K-259-2 in einem geeigneten Kulturmedium befähigt ist, und K-259-2 aus diesem Medium gewinnt.

**4.** Verfahren nach Anspruch 3, worin der Mikroorganismus Micromonospora sp. K-259 [FERM BP 569], oder eine zur Erzeugung von gemäß Anspruch 1 definiertem K-259-2 befähigte Mutante davon ist.

**5.** Stamm Mikromonospora sp. K-259 [FERM BP 569], und Mutanten davon, die zur Produktion von gemäß Anspruch 1 definiertem K-259-2 befähigt sind, wobei der Stamm biologisch rein ist.

**6.** Vasodilatorisches Mittel zur Anwendung bei Mensch oder Tier, welches eine wirksame Menge einer Substanz nach Anspruch 1 und einen physiologisch verträglichen Träger, Exzipienten oder Hilfsstoff enthält.

**7.** Verwendung einer Substanz nach Anspruch 1 zur Herstellung eines vasodilatorischen Mittels zur Anwendung bei Mensch oder Tier.

Fig. 1

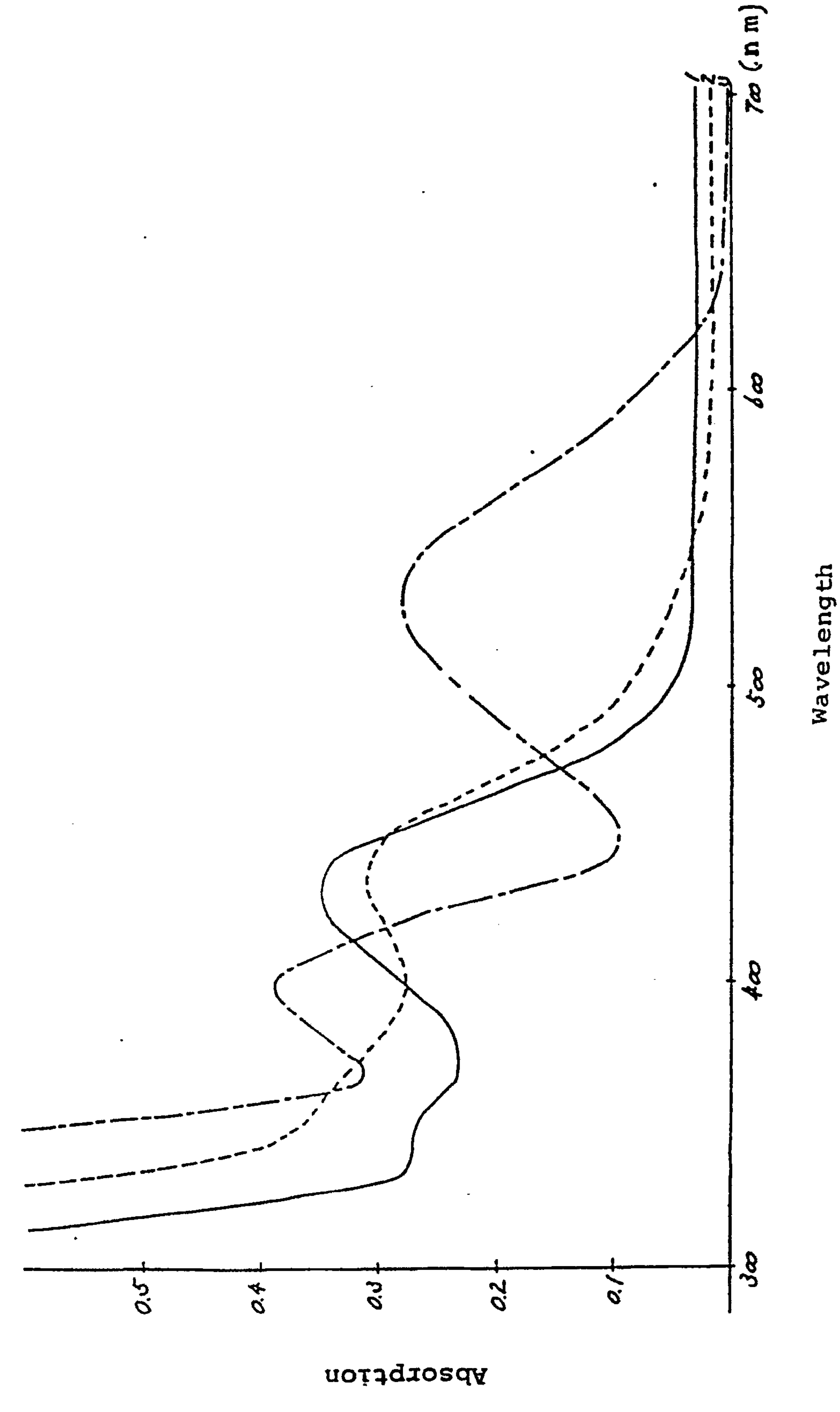
0.5
0.4
0.3
0.2
0.1
Absorption
300
400
500
600
700 (nm)
Wavelength
1
2
3

Fig. 2